(19) Europäisches Patentamt
European Patent Office
Office européen des brevets

(11) Veröffentlichungsnummer: **0 289 840**
**B1**

# (12) EUROPÄISCHE PATENTSCHRIFT

(45) Veröffentlichungstag der Patentschrift: **17.10.90**

(51) Int. Cl.⁵: **C07C 265/14**, C07C 263/10

(21) Anmeldenummer: **88106111.3**

(22) Anmeldetag: **16.04.88**

## (54) Verfahren zur Herstellung von (cyclo) aliphatischen Diisocyanaten.

(30) Priorität: **30.04.87 DE 3714439**

(43) Veröffentlichungstag der Anmeldung: **09.11.88 Patentblatt 88/45**

(45) Bekanntmachung des Hinweises auf die Patenterteilung: **17.10.90 Patentblatt 90/42**

(84) Benannte Vertragsstaaten: **BE DE ES FR GB IT NL**

(56) Entgegenhaltungen:
**DE-C- 870 847**
**GB-A- 656 726**
**GB-A- 1 165 831**

(73) Patentinhaber: **BAYER AG, D-5090 Leverkusen 1 Bayerwerk(DE)**

(72) Erfinder: **Frosch, Hans-Georg, Dr., Rotdornweg 29, D-5000 Köln 91(DE)**
Erfinder: **Grave, Heinrich, Dr., Haydnstrasse 4b, D-5060 Bergisch Gladbach 1(DE)**
Erfinder: **Stutz, Herbert, Dr., Schulstrasse 81, D-4047 Dormagen 5(DE)**
Erfinder: **Waldau, Eckart, Dr., Benrather Schlossufer 7, D-4000 Duesseldorf 13(DE)**
Erfinder: **Fuhrmann, Peter, Amandusstrasse 5, D-5000 Köln 71(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von (cyclo)aliphatischen Diisocyanaten durch Phosgenierung von (cyclo)alophatischen Diaminen in der Gasphase.

Die Herstellung von Isocyanaten durch Umsetzung von Aminen mit Phosgen in der Gasphase ist seit langem bekannt (vgl. Siefken, Annalen 562, 108 (1949)). Technische Bedeutung hat die Gasphasenphosgenierung jedoch nur bei der Umsetzung von Monoaminen gefunden, weil polyfunktionelle Amine sich zum Teil bei der Verdampfung zersetzen und während der Phosgenierung zur Bildung von Polymeren neigen (vgl. Ullman, 4. Auflage, Bd. 13, S. 353), was in der Regel zu niedrigen Ausbeuten führt.

So wird in der DE-PS 870 847 ein Verfahren beschrieben, bei dem Hexamethylendiamin in Gegenwart von Stickstoff und Benzol mit Phosgen in der Gasphase zu Hexamethylendiisocyanat umgesetzt wird. Es wird eine etwa 10 bis 15 %ige Ausbeute der Theorie erreicht. Diese unbefriedigende Ausbeute ist ein Nachteil des Verfahrens.

In zwei Veröffentlichungen (J. Chem. Soc. Japan, Ind. Chem. Sect. 55, 266-7 (1952) und ibid. 56, 289-90 (1953)) wird die Gasphasensynthese von Isocyanaten beschrieben, bei der das Amin zusammen mit Toluol ver-dampft wird und in der Gasphase in einem Rohrreaktor bei Temperaturen von maximal 325°C mit einem stöchiometrischen Überschuß an Phosgen von 130 % der Theorie zur Reaktion gebracht wird. Das dampfförmige Isocyanat wird in Toluol kondensiert. Hierbei setzen sich Teile des Isocyanats mit Chlorwasserstoff zu festem Carbamidsäurechlorid um. Zur Überführung des Carbamidsäurechlorids in das gewünschte Isocyanat wird die Suspension des Carbamidsäurechlorid in Toluol so lange unter Rückfluß erhitzt, bis kein Chlorwasserstoff mehr entweicht. Bei der Umsetzung von Hexamethylendiamin nach diesem Verfahren bei 280°C bis 300°C wird eine Ausbeute von 80 % erzielt. Nachteile dieses Verfahrens sind die niedrige Ausbeute und die kostenintensive thermische Spaltung des Carbamidsäurechlorids. Beide Nachteile lassen dieses Verfahren unwirtschaftlich erscheinen.

In der GB-PS 1 165 831 wird u.a. auch ein Verfahren der Gasphasenphogenierung von Diisocyanaten beschrieben, bei dem die Reaktion des dampfförmigen Amins mit dem Phosgen bei Temperaturen zwischen 150°C und 300°C in einem mit einem mechanischen Rührer ausgestatteten Rohrreaktor durchgeführt wird. Der Reaktor ähnelt einem Dünnschichtverdampfer, bei dem der Rührer die Gase mischt und gleichzeitig die beheizten Wände des Rohrreaktors bestreicht, um so ein Aufbau von polymerem Material an der Rohrwand zu verhindern. Feststoffe würden den Wärmeübergang erschweren und zum Abbruch der Reaktion führen. Die Verwendung eines mit etwa 1000 UpM laufenden Rührers beim Umgang mit ca. 300°C heißem Phosgen erfordert jedoch hohen sicherheitstechnischen Aufwand, um den Reaktor abzudichten und den Rührer in dem hochkorrosivem Medium zu lagern.

Wie jetzt überraschenderweise gefunden wurde, ist es möglich auch (cyclo)aliphatische Diisocyanate in hohen Ausbeuten durch Gasphasenphosgenierung der ihnen zugrundeliegenden Diamine unter Eliminierung der genannten Nachteile der Verfahren des Standes der Technik herzustellen, wenn die Umsetzung unter Beachtung bestimmter, nachstehend näher beschriebener Verfahrensparameter durchgeführt und das bei der Umsetzung resultierende Gasgemisch auf die nachstehend näher erläuterte Weise weiter aufgearbeitet wird.

Gegenstand der Erfindung ist ein Verfahren zur Herstellung von Diisocyanaten der allgemeinen Formel

OCN-R-NCO

in welcher
R für einen (cyclo)aliphatischen Kohlenwasserstoffrest mit bis zu 15 Kohlenstoffatomen steht, durch Phosgenierung der entsprechenden Diamine der allgemeinen Formel

$H_2N$-R-$NH_2$

in der Gasphase, dadurch gekenzeichnet, dab man

a) die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem auf 200°C bis 600°C erhitzten, zylindrischen Reaktionsraum ohne sich bewegende Teile unter Aufrechterhaltung einer turbulenten Strömung im Reaktionsraum miteinander zur Reaktion bringt,

b) das den Reaktionsraum kontinuierlich verlassende Gasgemisch durch ein inertes Lösungsmittel leitet, welches bei einer Temperatur oberhalb der Zersetzungstemperatur des dem Diamin entsprechenden Carbamidsäurechlorids gehalten wird, und

c) das in dem inerten Lösungsmittel gelöst vorliegende Diisocyanat einer destillativen Aufarbeitung unterzieht.

Ausgangsmaterialien für das erfindungsgemäße Verfahren sind (cyclo)aliphatische Diamine der allgemeinen Formel

$H_2N$-R-$NH_2$

in welcher
R für einen (cyclo)aliphatischen Kohlenwasserstoffrest mit bis zu 15, vorzugsweise 4 bis 13 Kohlenstoffatomen steht, mit der Maßgabe, daß zwischen den beiden Aminogruppen mindestens zwei Kohlenstoffatome angeordnet sind.

Unter einem "(cyclo)aliphatischen" Rest sind hierbei sowohl aliphatische als auch cycloaliphatische als auch aliphatisch-cycloaliphatische Reste zu verstehen, wobei sich "aliphatisch" bzw. "cycloaliphatisch" jeweils auf die Natur des mit den Aminogruppen verknüpften Kohlenstoffatoms bezieht.

Typische Beispiele geeigneter Diamine sind 1,4-Diaminobutan, 1,6-Diaminohexan, 1,11-Diaminoundecan, 1,4-Diaminocyclohexan, 1-Amino-3,3,5-trimethyl-5-aminomethylcyclohexan (IPDA), 4,4'-Diaminodicyclohexylmethan oder 4,4'-Diamino-dicyclohexylpropan-(2,2). Bevorzugte Ausgangsdiamine sind 1,6-Diaminohexan, IPDA und 4,4'-Diaminodicyclohexylmethan.

Die Ausgangsdiamine werden vor der Durchführung des erfindungsgemäßen Verfahrens verdampft und kontinuierlich auf eine Temperatur innerhalb des Temperaturbereichs von 200 bis 600°C, vorzugsweise 300 bis 500°C erhitzt. Die zuvor erhitzten Diamindämpfe können beim erfindungsge mäßen Verfahren als solche oder in mit Inertgas oder mit Dämpfen eines inerten Lösungsmittels verdünnter Form zum Einsatz gelangen. Die Durchmischung der Diamindämpfe mit dem Inertgas kann beispielsweise durch Verdampfen des Diamins im Strom eines inerten Gases oder der Dämpfe eines inerten Lösungsmittels erfolgen. Bevorzugtes Inertgas ist Stickstoff. Geeignete inerte Lösungsmittel, deren Dämpfe ebenfalls zur Verdünnung des Diamins verwendet werden können sind beispielsweise Chlorbenzol, o-Dichlorbenzol, Xylol, Chlornaphthalin, Decahydronaphthalin oder deren Gemische.

Die Menge des gegebenenfalls als Verdünnungsmittel mitverwendeten Inertgases oder Lösungsmitteldampfes ist nicht kritisch. Falls eine Verdünnung des Diamins stattfindet, kann dies beispielsweise unter Einhaltung eines Volumenverhältnisses von Diamindampf zu Inertgas bzw. Lösungsmitteldampf von 1:0,5 bis 1:2 erfolgen.

Das bei der Phosgenierung verwendete Phosgen wird, bezogen auf das Diamin, im Überschuß eingesetzt. Im allgemeinen genügt eine Phosgenmenge, die 150 bis 250 % der Theorie bezüglich der ablaufenden Phosgenierungsreaktion entspricht.

Vor der Durchführung des erfindungsgemäßen Verfahrens wird der Phosgenstrom auf eine Temperatur innerhalb des Bereichs von 200 bis 600°C, vorzugsweise 300 bis 500°C erhitzt.

Zur Durchführung der erfindungsgemäßen Umsetzung werden die vorerhitzten Ströme von Diamin bzw. Diamin-Inertgas-Gemisch einerseits von Phosgen andererseits kontinuierlich in einen zylindrischen Reaktionsraum geleitet und dort miteinander vermischt.

Geeignete zylinderförmige Reaktionsräume sind beispielsweise Rohrreaktoren ohne Einbauten und ohne sich bewegende Teile im Innern des Reaktors. Die Rohrreaktoren bestehen im allgemeinen aus Stahl, Glas, legiertem oder emailliertem Stahl und weisen eine Länge auf, die ausreichend ist, um unter den Verfahrensbedingungen eine vollständige Umsetzung des Diamins mit dem Phosgen zu ermöglichen. Die Gasströme werden im allgemeinen an einem Ende des Rohrreaktors in diesen eingeleitet, wobei diese Einleitung beispielsweise durch an einem Ende des Rohrreaktors angebrachte Düsen oder durch eine Kombination aus Düse und einem Ringspalt zwischen Düse und Mischrohr erfolgen kann. Das Mischrohr wird ebenfalls bei einer Temperatur innerhalb des Bereichs von 200 bis 600°C, vorzugsweise 300 bis 500°C gehalten, wobei diese Temperatur gegebenenfalls durch Beheizen des Reaktionsrohrs aufrechterhalten wird.

Für die Durchführbarkeit des erfindungsgemäßen Verfahrens wesentlich ist, daß die Abmessungen des Rohrreaktors und die Strömungsgeschwindigkeiten im Reaktionsraum so bemessen werden, daß in dem Reaktionsraum eine turbulente Strömung herrscht. Unter "turbulenter Strömung" ist hierbei eine Reynoldszahl von mindestens 2500, vorzugsweise mindestens 4700 zu verstehen. Im allgemeinen ist diese Turbulenz im Reaktionsraum dann gewährleistet, wenn die gasförmigen Reaktionspartner mit einer Strömungsgeschwindigkeit von mehr als 90 m/s den Reaktionsraum durchlaufen. Diese Strömungsgeschwindigkeit kann durch Einstellung eines entsprechenden Differenzdrucks zwischen den Produktzuleitungen zum Reaktionsraum einerseits und Ausgang aus dem Reaktionsraum andererseits sichergestellt werden. Im allgemeinen liegt der Druck in den Zuleitungen zum Reaktionsraum bei 200 bis 3000 mbar und am Ausgang aus dem Reaktionsraum bei 150 bis 2000 mbar. Wesentlich ist hierbei jedoch lediglich die Aufrechterhaltung eines Differenzdrucks zwecks Gewährleistung der genannten Strömungsgeschwindigkeit.

Nach der erfolgten Phosgenierungsreaktion im Reaktionsraum wird das den Reaktionsraum kontinuierlich verlassende, gasförmige Gemisch von dem gebildeten Diisocyanat befreit. Dies kann beispielsweise durch selektive Kondensation in einem inerten Lösungsmittel erfolgen. Die Temperatur des Lösungsmittels wird dabei so gewählt, daß sie einerseits oberhalb der Zersetzungstemperatur des dem Diisocyanat entsprechenden Carbamidsäurechlorids liegt und andererseits das Diisocyanat und gegebenenfalls das in der Dampfform als Verdünnungsmittel mitverwendete Lösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des Diisocyanats aus dem, den Reaktionsraum gasförmig verlassenden Gemisch besonders gut geeignet sind bei einer Temperatur von 120 bis 200°C, vorzugsweise 120 bis 170°C gehaltene Lösungsmittel der oben beispielhaft genannten Art, insbesondere technisches Dichlorbenzol.

Das die Kondensationsstufe zur Gewinnung des Diisocyanats durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol oder Dichlorbenzol) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recyclisiert werden.

Die Reindarstellung der Diisocyanate erfolgt durch destillative Aufarbeitung der Lösung des Diisocyanats in dem zur Diisocyanatkondensation eingesetzten Lösungsmittel.

In den nachfolgenden Beispielen beziehen sich alle Prozentangaben auf Gewichtsprozente.

Beispiel 1

In ein auf 400°C erwärmtes Mischrohr von 2,5 mm Durchmesser und 17,5 mm Länge mit nachgeschalteter Diisocyanatkondensationsstufe und dieser nachfolgendem, mit Aktivkohle gefülltem Phosgen-Adsorptionsturm strömen kontinuierlich durch eine Düse, die in das Mischrohr hineinragt, 5,91 Mol/Stunde Phosgen, die in einem vorgeschalteten Wärmetauscher bei einem Druck von 1100 mbar auf eine Temperatur von 400°C erwärmt wurden. Durch den Ringspalt zwischen Düse und Mischrohr wird gleichzeitig ein auf 400°C erwärmtes Gemisch aus 1,26 Molen gasförmigen Hexamethylendiamin und 1,25 Molen Stickstoff stündlich in das Mischrohr geleitet. Durch Anlegen eines Vakuums am Ausgang aus dem Phosgen-Adsorptionsturm wird ein Druck im Mischrohr von ca. 350 mbar aufrechterhalten. In dem Reaktionsraum, der von den Gasen mit einer Geschwindigkeit von 190 m/s durchlaufen wird, herrscht eine turbulente Strömung (Reynolds-Zahl = 10000) vor. Das heiße, den Reaktionsraum gasförmig verlassende Reaktionsgemisch wird in der Kondensationsstufe durch Dichlorbenzol geleitet, welches bei einer Temperatur von 150 bis 160°C gehalten wird. Hierbei erfolgt eine selektive Kondensation des gebildeten Diisocyanatohexans. Das die Waschstufe durchlaufende, im wesentlichen aus Stickstoff, Chlorwasserstoff und überschüssigem Phosgen bestehende Gasgemisch wird in dem Adsorptionsturm anschließend vom Phosgen befreit. Das Diisocyanat wird aus dem Waschlösungsmittel destillativ in reiner Form gewonnen. Die Ausbeute an 1,6-Diisocyanatohexan liegt bei 98,0 % der Theorie.

Beispiel 2

Unter den Verfahrensbedingungen des Beispiels 1 werden 1,26 Mol/Stunde 1-Amino-3,3,5-trimethyl-5-aminomethylcyclohexan mit 5,91 Mol/Stunde Phosgen unter Mitverwendung von 1,25 Mol/Stunde Stickstoff als Verdünnungsmittel für das Diamin in 99,9 %iger Ausbeute in 1-Isocyanato-3,3,5-trimethyl-5-isocyanatomethyl-cyclohexan überführt.

Beispiel 3

Unter den in Beispiel 1 genannten Reaktionsbedingungen werden 1,26 Mol/Stunde 4,4′-Diaminodicyclohexylmethan mit 5,91 Mol/Stunde Phosgen unter Mitverwendung von 1,25 Mol/Stunde Stickstoff als Verdünnungsmittel für das Diamin in 97,8 %iger Ausbeute in 4,4′-Diisocyanatodicyclohexylmethan überführt.

**Patentansprüche**

1. Verfahren zur Herstellung von Diisocyanaten der allgemeinen Formel

OCN-R-NCO

in welcher
R für einen (cyclo)aliphatischen Kohlenwasserstoffrest mit bis zu 15 Kohlenstoffatomen steht,
durch Phosgenierung der entsprechenden Diamine der allgemeinen Formel

$H_2N-R-NH_2$

in der Gasphase, dadurch gekennzeichnet, daß man
a) die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und kontinuierlich in einem auf 200°C bis 600°C erhitzten, zylindrischen Reaktionsraum ohne sich bewegende Teile unter Aufrechterhaltung einer turbulenten Strömung im Reaktionsraum miteinander zur Reaktion bringt,
b) das den Reaktionsraum kontinuierlich verlassende Gasgemisch durch ein inertes Lösungsmittel leitet, welches bei einer Temperatur oberhalb der Zersetzungstemperatur des dem Diamin entsprechenden Carbamidsäurechlorids gehalten wird, und
c) das in dem inerten Lösungsmittel gelöst vorliegende Diisocyanat einer destillativen Aufarbeitung unterzieht.

2. Verfahren gemäß Anspruch 1, dadurch gekennzeichnet, daß die in dem Reaktionsraum vorherrschende Turbulenz einer Reynoldszahl von mindestens 2500 entspricht.

3. Verfahren gemäß Anspruch 1 und 2, dadurch gekennzeichnet, daß man als phosgenierende Diamine 1,6-Diaminohexan, 1-Amino-3,3,5-trimethyl-5-aminomethyl-cyclohexan oder 4,4′-Diaminodicyclohexylmethan verwendet.

4. Verfahren gemäß Anspruch 1 bis 3, dadurch gekennzeichnet, daß man die Umsetzung in dem Reaktionsraum bei einer Temperatur von 300 bis 500°C durchführt.

5. Verfahren gemäß Anspruch 1 bis 4, dadurch gekennzeichnet, daß man als Inertgas zur Verdünnung des dampfförmigen Diamins Stickstoff verwendet, wobei das Volumenverhältnis von Diamin zu Stickstoff bei 1:0,5 bis 1:2 liegt.

**Claims**

1. A process for the production of diisocyanates corresponding to the following general formula

OCN-R-NCO

in which
R is a (cyclo)aliphatic hydrocarbon radical containing up to 15 carbon atoms, by phosgenation of the corresponding diamines corresponding to the following general formula

$H_2N-R-NH_2$

in the gas phase, characterized in that
a) the vaporous diamines, optionally diluted with an inert gas or with the vapours of an inert solvent, and phosgene are separately heated to temperatures of 200°C to 600°C and continuously reacted with one another in a cylindrical reaction zone with no moving parts which has been heated to 200 to 600°C, a turbulent flow being maintained in the reaction zone,

b) the gas mixture continuously leaving the reaction zone is passed through an inert solvent which is kept at a temperature above the decomposition temperature of the carbamic acid chloride corresponding to the diamine and

c) the diisocyanate dissolved in the inert solvent is worked up by distillation.

2. A process as claimed in claim 1, characterized in that the turbulence prevailing in the reaction zone corresponds to a Reynolds number of at least 2,500.

3. A process as claimed in claims 1 and 2, characterized in that 1,6-diaminohexane, 1-amino-3,3,5-trimethyl-5aminomethyl cyclohexane or 4,4'-diaminodicyclohexyl methane is used as the diamine to be phosgenated.

4. A process as claimed in claims 1 to 3, characterized in that the reaction is carried out at a temperature of 300 to 500°C in the reaction zone.

5. A process as claimed in claims 1 to 4, characterized in that nitrogen is used as the inert gas for diluting the vaporous diamine, the ratio by volume of diamine to nitrogen being from 1:0.5 to 1:2.

## Revendications

1. Procédé pour la fabrication de diisocyanates de formule générale

OCN-R-NCO

dans laquelle
R représente un reste (cyclo)aliphatique hydrocarboné possédant jusqu'à 15 atomes de carbone, par phosgénation de la diamine correspondante de formule générale

$H_2N$-R-$NH_2$

en phase gazeuse, caractérisé en ce que

a) l'on chauffe séparément à des températures de 200°C à 600°C la diamine à l'état de vapeur, éventuellement diluée avec un gaz inerte ou avec les vapeurs d'un solvant inerte, et le phosgène et qu'on les fait réagir ensemble de façon continue dans un espace de réaction cylindrique chauffé à une température de 200°C à 600°C ne comportant pas de pièces en mouvement, en maintenant un écoulement turbulent dans 1, espace de réaction,

b) l'on fait passer le mélange gazeux quittant continuellement l'espace de réaction par un solvant inerte maintenu à une température supérieure à la température de décomposition du chlorure d'acide carbamique correspondant à la diamine, et

c) l'on soumet le diisocyanate présent à 1, état dissous dans le solvant inerte à un traitement par distillation.

2. Procédé selon la revendication 1, caractérisé en ce que la turbulence régnant dans l'espace de réaction correspond à un nombre de Reynolds d'au moins 2500.

3. Procédé selon la revendication 1 ou 2, caractérisé en ce que l'on utilise comme diamine à soumettre à la phosgénation le 1,6-diamino-hexane, le 1-amino-3,3 5-triméthyl-5-amino-méthyl-cyclohexane ou le 4,4'-diamino-dicyclohexylméthane.

4. Procédé selon les revendications 1 à 3, caractérisé en ce que 1, on effectue la réaction dans un espace de réaction à une température de 300 à 500°C.

5. Procédé selon les revendications 1 à 4, caractérisé en ce que l'on utilise l'azote comme gaz inerte pour la dilution de la diamine à l'état de vapeur, le rapport en volume entre la diamine et l'azote étant de 1:0, 5 à 1:2.